(19) Europäisches Patentamt
European Patent Office
Office européen des brevets

(11) Publication number: **0 343 946 A2**

(12) # EUROPEAN PATENT APPLICATION

(21) Application number: **89305232.4**

(22) Date of filing: **24.05.89**

(51) Int. Cl.4: **C 07 K 7/10**
C 07 H 21/04, C 12 N 15/00

(30) Priority: **25.05.88 US 198289**

(43) Date of publication of application:
**29.11.89 Bulletin 89/48**

(84) Designated Contracting States: **DE FR GB**

(71) Applicant: **CITY OF HOPE**
**1500 East Duarte Road**
**Duarte California 91010-0269 (US)**

(72) Inventor: **Shively, John Ernest**
**1657 Wilson**
**Arcadia California 91006 (US)**

(74) Representative: **Hallybone, Huw George et al**
**CARPMAELS AND RANSFORD 43 Bloomsbury Square**
**London WC1A 2RA (GB)**

(54) **Cea fragments.**

(57) Synthetic CEA fragments that include a unique epitope, assays which utilize such fragments to detect and quantify CEA in samples and kits useful to conduct such assays.

EP 0 343 946 A2

Description

# CEA FRAGMENTS

## BACKGROUND OF THE INVENTION

Carcinoembryonic antigen (CEA) is a 180-Kda glycosylated glycoprotein. It was described in 1965 as a tumor marker for colonic cancer.

Immunological and biochemical studies have demonstrated that CEA is not tumor specific, but instead only one of several closely related antigens which share common epitopes. Some of the normal occurring related antigens have unique epitopes which permit discrimination from CEA. Others may interfere with the determination of CEA in serum and tissue samples.1/ Extensive sequence homology places CEA, its normal cross-reacting antigen (NCA),2/ and a more recently discovered 128 Kda antigen3/ within the immunoglobulin supergene family.4/ Distinguishing antisera are difficult to obtain.

Diagrams 1A, 1B and 1C are linear domain models of NCA, the 128 Kda antigen and CEA, each of which is three dimensional in space.5/

---

1/ See, e.g., Tawaragi, et al., (1987) Biochem. and Biophys. Res. Commun. 150, 89; Neumaier, et al., (1985) Mol. Immunol. 22, 1273-1277; Neumaier, M., et al., (1985) J. Immunol. 135, 3604-3609; Svenberg, T. (1976) Int. J. Cancer 17, 588-596; and Burtin, P., et al., (1973) J. Immunol. 111, 1926-1928.

2/ See, e.g., Tawaragi, supra, n. 1; Buchegger, F., et al., (1984) Int. J. Cancer 33, 643-649; von Kleist, S., et al., (1972) Proc. Natl. Acad. Sci. U.S.A. 69, 2492-2494; and Mach, J.-P., et al., (1972), Immunochemistry 9, 1031-1034.

3/ See, e.g., Neumaier, et al., (1985) J. Immunol. 135:3604-3609, supra, n. 1.

4/ See, as to CEA and NCA, Paxton R., et al., (1987) Proc. Natl. Acad. Sci. (U.S.A.) 84 920-924.

---

5/ In each of the 1A, 1B and 1C Diagrams, the "N" domains are an N-terminal sequence, and the "M" domains are hydrophobic carboxy terminal sequences. Three Ig like domains, each with A and B subdomains, are labelled "I", "II" and "III". Signal sequences which precede the amino terminus of the "N" domain are not present in mature proteins and hence are omitted from the diagrams.

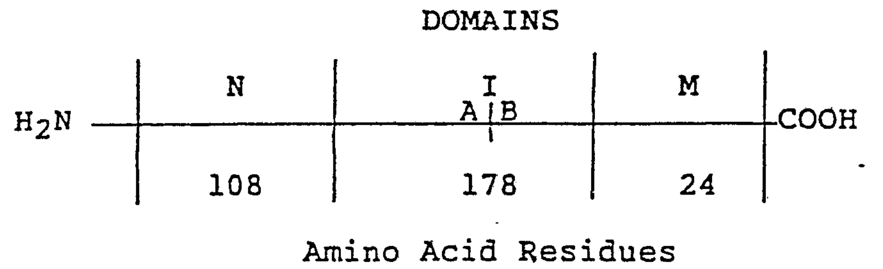


DIAGRAM 1A—NCA DOMAIN MODEL

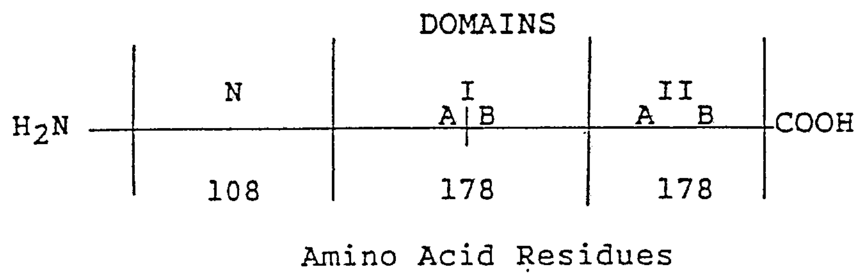


DIAGRAM 1B—128 Kda ANTIGEN

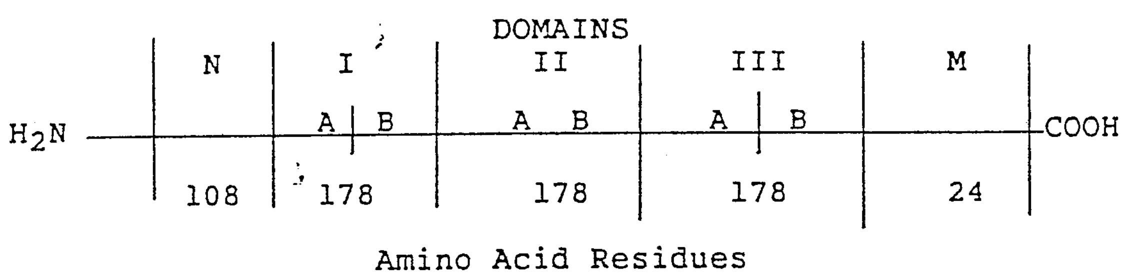


DIAGRAM 1C—CEA

As Diagrams 1A, 1B and 1C show, NCA contains only one Ig like 178 residue domain (I), the 128 Kda contains two such domains (I and II) and CEA contains three such domains (I, II, and III).

The 128 Kda antigen is presumed to be a proteolytic fragment of CEA. Amino acid sequence studies show that the domains N, I, and II are present, and that the amino acid sequences are identical to CEA. Domain III is absent. Protein structural studies on CEA and NCA also reveal that the M domains predicted by the cDNA sequences are absent in the mature proteins. In the case of CEA, this domain is replaced by a phosphinositol glycan moeity. The same may be true for NCA.

The subdomains A and B included in each of the domains I, II, and III include 92 and 86 amino acid residues respectively.6/

6/ See (1987) Oikawa, Kosaki and Nakazato, Biochem. and Biophys. Res. Commun. 14, 464 and Tawaragi, supra, n. 2.

Sequence I sets forth the nucleotide and amino acid sequences of the CEA domain III with a designation of the subdomains.

```
5' GAGCTGCCCAAGCCCTCCATCTCCAGCAACAACTCCAAACCCGTGGAG
   GluLeuProLysProSerIleSerSerAsnAsnSerLysProValGlu
                       470                        480

   GACAAGGATGCTGTGGCCTTCACCTGTGAACCTGAGGCTCAGAACACAACCTACCTGTGG
   AspLysAspAlaValAlaPheThrCysGluProGluAlaGlnAsnThrThrTyrLeuTrp
                                490                         500

   TGGGTAAATGGTCAGAGCCTCCCAGTCAGTCCCAGGCTGCAGCTGTCCAATGGCAACAGG
   TrpValAsnGlyGlnSerLeuProValSerProArgLeuGlnLeuSerAsnGlyAsnArg
                                510                         520

   ACCCTCACTCTATTCAATGTCACAAGAAATGACGCAAGAGCCTATGTATGTGGAATCCAG
   ThrLeuThrLeuPheAsnValThrArgAsnAspAlaArgAlaTyrValCysGlyIleGln
                                530                         540

                       <------DOMAIN A        ----DOMAIN B --->
   AACTCAGTGAGTGCAAACCGCAGTGACCCAGTCACCCTGGATGTCCTCTATGGCCGGAC
   AsnSerValSerAlaAsnArgSerAspProValThrLeuAspValLeuTyrGlyProAsp
                                550                         560

   ACCCCCATCATTTCCCCCCCAGACTCGTCTTACCTTTCGGGAGCGAACCTCAACCTCTCC
   ThrProIleIleSerProProAspSerSerTyrLeuSerGlyAlaAsnLeuAsnLeuSer
                                570                         580

   TGCCACTCGGCCTCTAACCCATCCCCGCAGTATTCTTGGCGTATCAATGGGATACCGCAG
   CysHisSerAlaSerAsnProSerProGlnTyrSerTrpArgIleAsnGlyIleProGln
                                590                         600

   CAACACACACAAGTTCTCTTTATCGCCAAAATCACGCCAAATAATAACGGGACCTATGCC
   GlnHisThrGlnValLeuPheIleAlaLysIleThrProAsnAsnAsnGlyThrTyrAla
                                610                         620

   TGTTTTGTCTCTAACTTGGCTACTGGCCGCAATAATTCCATAGTCAAGAGCATCACAGTC
   CysPheValSerAsnLeuAlaThrGlyArgAsnAsnSerIleValLysSerIleThrVal
                                630                         640

   TCTGCA
   SerAla 3'7/          SEQUENCE I
```

## SUMMARY OF THE INVENTION

Neumaier, et al.8/ demonstrated that the antibody T84.66-A3.1-H11 (ATCC Accession No. HB8747) (T84.66) reacts with CEA and not with the 128 Kda antigen. Because CEA and the 128 Kda antigen are otherwise identical, it is apparent that domain III includes the unique T84.66 epitope. The invention accordingly subsumes various natural and synthetic, preferably substantially pure, CEA fragments that contain that epitope, assays which utilize such fragments to determine the presence and amount of CEA in samples which may also contain related antigens including NCA or the 128 Kda antigen and to kits useful to conduct such assays.

---

7/ For the sequence information set forth in Figure 1, see (1986) Oikawa, et al., Biochem. and Biophys. Res. Commun. 142 511 (Fig. 2B, p. 515).

8/ Neumaier, et al., (1985) Mol. Immunol. 22, 1273-127.

## DETAILED DESCRIPTION OF THE INVENTION

### Topology of CEA Domain III

Each corresponding domain shows extensive homology between NCA, the 128 Kda antigen and CEA. Homology is 85% at the amino acid level and 95% at the nucleotide level for the N domains. A comparison of the homologies at the nucleotide level in the three copies of the Ig like repeated 178 residue in CEA domains I, II and III to the double copy of the same residue in the 128 Kda antigen domains I and II and the single copy for NCA domain I reveals the highest homology for the first copy (90%) compared to 83 to 86% for the second and third copies respectively.

This high degree of homology together with previous observations that NCA, the 128 Kda antigen and CEA are members of the Ig supergene family, has lead to the further suggestion that CEA may fold like adjacent domains of immunoglobulin.

Each copy of the repeat domains I, II and III contains two Ig like subdomains A and B. These subdomains are analogous to adjacent immunoglobulin domains CH2 and CH3 and would be similarly interactive and likely to form dimeric two-chain structures. A further indication that the folding pattern of CEA is indeed like Ig, is that the glycosylation sites appear primarily on the surface of the molecule9/ or at the ends of the β sheets and so preclude undesired interaction and interference with folding.

Figure 1 depicts the topology of the constant region of the CH-1 domain of the heavy chain of human immunoglobulin.10/

Figures 2A, 3A and 4A illustrate the topology of the A subdomains of the CEA domains I, II and III.

Figures 2B, 3B and 4B illustrate the topology of the B subdomains of the CEA domains I, II, and III.

In each of Figures 2A and B through 4A and B, the letters A, B, C, D, E, F, and G identify β sheets corresponding to the β sheets of the human immoglobulin domain depicted by Figure 1. Glycosylation sites are indicated by the symbol

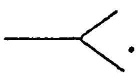

Similarly positioned disulfide bonds between β sheets C and F are shown in each Figure.

As the Figures show, the glycosylation sites on each of the A and B subdomains of CEA domains I, II and III appear primarily on the loops or at the β sheet ends and hence on the molecular surface.

This invention accordingly includes CEA fragments of Ig-like topology comprising nucleotide and amino acid sequences which substantially correspond to each of CEA domains IIIA and IIIB.

A comparison of the amino acid sequences in the CEA subdomains A and B and in the light and heavy Ig chains also evidences similarity of the two molecules. Tables 1 and 2 organize the amino acid sequence of CEA beginning with domain 1A (residue 108) into predicted β-strands (bold) and interconnecting loops (normal type). Thus the sequence continues from top to bottom in column 1A (Table 1) to column 1B (Table 2) to column 2A (Table a) to column 2B (Table 2) to column 3A (Table 1) to column 3B (Table 2). For comparison, the CH2 and CH3 domains of an immoglobulin (ref. 9) are shown.

Table 1 compares the predicted β sheet and interconnecting loop amino acid sequences of the A subdomain of each of CEA domains I, II and III with the amino acid sequences of the IgG domain $CH_3$. β sheet sequences are in bold face. The predictions were made by the method of Chou and Fasman, Biochemistry 13:222-224 (1974)..

---

9/ See Oikawa, et al. (1987) Biochem. Biophys. Res. Commun. 142 (634-642) and Neumaier, M., et al., (1985) J. Immunol. 135, 3604-3609, supra, n. 1.

---

10/ Neumaier, et al. (1988) J. Biol. Chem. 263:3202-3207 (1988); Atlas of Protein Sequence and Structure, National Biomedical Research Foundation, 1978, Vol. 5 Supplement 3, 212, Georgetown University Medical Center, Washington, D.C. 20007.

TABLE 1

| CEA | | | IgG | |
|---|---|---|---|---|
| REPEAT 1A | REPEAT 2A | REPEAT 3A | CH2 | CH3 |
| PELPKP | VYAEPPKP | VSAELPKP | IFPPKP | PPTVYPLAP |
| SISSNNSK | FITSNNSN | SISSNNSK | KDTLLITVT | GSNAASQ |
| PVED | PVED | PVED | PK | SM |
| KDAVAFTCE | EDAVALTCE | KDAVAFTCE | VTCVVVDISK | VTLGCLVKGYF |
| PETQ | PEIQ | PEAN | DDP | PEP |
| DATYLWWV | NTTYLWWV | TTYLWWV | EVQFSWF | VTVTWNS |
| NNQSLPVSP | NNQSLPVSP | NGQSLPVSP | VDNVE | GSLSSG |
| RLQLSN | RLQLSN | RLQLSN | VHTAQTQ | VHTFPAVL |
| GRN | DNR | GNR | PREEQ | QSDLY |
| TLTLFNVTR | TLTLLSVTR | TLTLFNVTR | FNSTFRVSAL | TLSSSVSV |
| NDTSA | NDVGR | NDARA | PIMHQPWLNGK | PTSPE |
| YKCETQN | YECGIQN | YVCGIQN | EFKCRVNS | TVTCNVA |
| PNSA | ELSV | SVSA | PAP | HAP |
| RRSDSVIL | DHSDPVIL | NRSDPVTL | IEKTISKTK | SSTKVDKK |

Table 2 compares the β sheet and loop amino acid sequences of the B subdomain of each of CEA domains I, II, and III with the amino acid sequences of the IgG domains CH1 and CH2. β sheet sequences are in bold face.

TABLE 2[11/]

| CEA | | | IgG |
|---|---|---|---|
| REPEAT 1B | REPEAT 2B | REPEAT 3B | CH3 |
| NVLYGPDAPTISP | NVLYGPDDPTISP | DVLYGPDTPIISPP | GKRPAPQVYTIPPP |
| LNTSYRSG | SYTTYRPG | DSSYLSG | KEQMAKDK |
| ENL | VNL | ANL | KV |
| NLSCHAASN | SLSCHAASN | NLSCHSASN | SLTCMITD |
| PPA | PPA | PSP | FFP |
| GYSWFVNG | QYSWLIDG | QYSWRING | EDITVEWQ |
| TFQ | NIQ | IPQ | SDGQAP |
| QSTQEL | QHTQEL | QHTQVL | ENYKNTQ |
| FIP | FIS | FIA | PIMDT |
| NITVNN | NITEKN | KITPNN | DGSYFVYSKLN |
| SGS | SGL | NGT | VQKSNWEAGN |
| YTCQAHNS | YTCQANNS | YACFVSNL | TFTCSVLH |
| DTGL | ASGH | ATGR | GLH |
| NRTTVTTIT | SRTTVKTIT | NNSIVKSIT | NNHTEKSLS |

The immonoglobulin molecule has a fold which includes seven β-sheets arranged in a 4-3 pattern (see Figure 3). The two groups of β-sheets are held together by a disulfide bond. Thus, one domain (e.g. CH2 or CH3) in an immunoglobulin has two cysteine residues engaged in a disulfide bond. The fact that CEA has its cysteine residues (heavy dot) placed in the identical β-sheets as CH2 or CH3 (in this model) is supportive evidence that this model for CEA (base on Ig) is correct.

11/ Amino acid residues are indicated by the first letter code convention. See (1988) J. Biol. Chem. 263 597.

An important part of the invention includes amino acid sequences which include the beta sheets and certain of the connecting loops of the A and B domains of CEA domain III. In particular, the invention includes synthetic peptides consisting of or comprising each of the following sequences:

II

KDAVAFTCEPETQDATYLWWVNNQSLPVSPRLQLSNGNRTLTLFNVTRNDTASYKCETQN (60) This sequence includes residues 126-186 from the CEA IA subdomain. It contains five β-strands B through F (Fig. 4a) and the disulfide bridge. It has twelve amino acid differences (underlined) from NCA.

III

EDAVALTCEPEIQNTTYLWWVNNQSLPVSPRLQLSNDNRTLTLLSVTRNDVGPYECGIQN (60) This sequence includes residues 304-363 from the CEA IIA subdomain. It is analogous to II. 13 amino acid differences from NCA are underlined.

IV

KDAVAFTCEPEAQNTTYLWWVNGQSLPVSPRLQLSNGNRTLTLFNVTRNDARAYVXGIQN (60) This sequence includes residues 482-541 from the CEA IIIA subdomain. It is analogous to II and III. 11 amino acid differences from NCA are underlined.

V

NLSCHAASNPPAQYSWFVNGTFQQSTQELFIPNITVNNSGSYTCQAHNS (49) This sequence includes residues 222-270 from the CEA IB subdomain. It contains five (5) β strands and four amino acid differences (underlined) from NCA.

VI

SLSCHAASNPPAQYSWLIDGNIQQHTQELFISNITEKNSGLYTCQANNS (49) This sequence includes residues 400-448 from the CEA IIB subdomain. Sequence is analogous to V. Includes second disulfide loop. 13 amino acid differences from NCA are underlined.

VII

NLSCHSASNPSPQYSWRINGIPQQHTQVLFIAK ITPNNNGTYACFVSNL (49)
This sequence includes residues 578-626 from the CEA IIIB subdomains It is analogous to V and VI. 18 amino acid differences from NCA are underlined.

## Preparation and Expression of CEA Fragments

The invention comprises natural or synthetic fragments of a CEA molecule which include one or more of each of the Ig like domains I, II and III; one or more of each of the subdomains IA, IB, IIA, IIB, IIIA and IIIB and the expression of such fragments, e.g., in a eukaryotic expression system.

Synthetic DNA coding for any desired CEA fragment including fragments encoding for any of the aforementioned peptide sequences II through VII is prepared in known manner by use, for example, of a Syntec microsyn 1450 automated DNA synthesizer and purified by polyacrylamide gel electrophoresis and high-performance liquid chromatography.

The cDNA sequence of CEA is known.12/ Synthetic restriction sites for separation of each of the various domains and subdomains may be provided by mutation of CEA in known manner.13/

Diagram 2 depicts an example of the introduction of restriction sites for separation at the junction of subdomains A and B of each of CEA domains I, II and III.

---

12/ See, e.g., Oikawa, S., et al., Biochem. Biophys. Res. Commun. 142:511-518 (1987) and Oikawa, S., et al, Biochem. Biophys. Res. Commun. 144:634-642 (1987).

13/ See, e.g., (1987) "Muta-Gene™ in Vitro Mutagenesis Kit Instruction Manual, Bio-Rad Laboratories, 1414 Harbor Way South, Richmond, CA 94804.

A-B DOMAIN JUNCTIONS

| | | | | | | | |
|---|---|---|---|---|---|---|---|
| | Asp | Ser | Val | Ile | Leu | Asn | Val |
| I. | GAT | TCA | GTC | ATC | CTG | AAT | GTC |
| | Asp | Pro | Val | Ile | Leu | Asn | Val |
| II. | GAC | CCA | GTC | ATC | CTG | AAT | GTC |
| | Asp | Pro | Val | Thr | Leu | Asp | Val |
| III. | GAC | CCA | GTC | ACC | CTG | GAT | GTC |
| | | | GTT | ACC | | | |

Hpal

DIAGRAM 2

In this example, a restriction site as close as possible to the domain junction is desired. The nucleotide sequences near the domain can be converted to a Hpa1 site by merely making each GTT ACC in the synthetic gene or by oligonucleotide directed mutagenesis in the natural gene. This change will preserve the Val residue for I, II, and III, but convert all three domains of the second residue into Thr (which it already is in III).

Like procedures are useful to provide mutant restriction sites between subdomains A and B of CEA domains I and II.

## Expression of CEA Molecular Fragments in Eukaryotic System

Known techniques provide eukaryotic expression of each of the natural and synthetic fragments of CEA molecules. The CEA gene fragments are inserted into eukaryotic expression vectors and transfected into appropriate cell lines, for example myeloma cell line such as SP20. The procedure is identical to that described for expressing immunogloublins in transfectomas. Examples are described by Morrison, et al., PNAS 81:6851-6855 (1984); Sun, et al., PNAS 84:214-218 (1987); Nishimura, et al., Cancer Res. 47:999-1005 (1987); Lu, et al., PNAS 84:3439-3443 (1987); Schnee, et al., PNAS 84:6904-6908 (1987); Brown, et al., Cancer Res. 47:3577-3583 (1987); Shaw, et al., J. Immunology 138:4534-4538 (1987); and Hones, et al., Nature 321:522=525 (1986). The vectors are derivatives of pSV2 with the neo or gpt genes to monitor transfection. Other vectors are commercially available, e.g., PEUK-C from Clon Tech.

For example, the pSV2 vector with gpt or neo genes described by Morrison, et. al, PNAS 81:6851 (1984) may have a CEA gene cloned into BamH1 sites provided by BamH1 linkers. The vector may be electroporated into Sp210 as described by Toneguzzo and Keating, PNAS 83:3496-3499 (1986). The cells may be grown with the appropriate antibotic to obtain stable transformants. These cells may be screened for the secretion production of CEA by an ELISA and by FACS for surface production.

Expression of other molecular fragments of CEA may be accomplished by similar procedures.

## Immunoassays

The molecular fragments of the invention are useful in any desired immunoassay procedure to discriminate between and quantify CEA present in a sample also containing NCA and other CEA-related antigens. This aspect of the invention is not limited to any particular assay procedures. Direct and indirect assay procedures may be utilized.

For use in such assays, the CEA fragments may be provided with any desired radio, fluorescent, enzymatic, or chemiluminescent label. Enzyme immunoassays (EIA), particularly non-competitive enzyme linked immunosorbent (ELISA) assays are preferred. Enzymes useful to label CEA fragments include, among others alkaline phosphatase, β-galactosidase, horseradish peroxidase, glucose-6-phosphate dehydrogenase, 3-phosphoglycerate kinase (PGK). Fusion proteins formed from CEA fragments and enzymes are appropriately prepared in known manner. See, e.g., the heavy chain TPA fusion protein described by Schnee,

et al., PNAS 84:6904-6908 (1987).

Enzymes already popular in EIAS e.g., alkaline phosphatase and β-galactosidase are preferred. Either the natural or synthetic genes can be used. The bacterial genes for both are. readily available.

Diagram 3 illustrates a typical fusion gene:

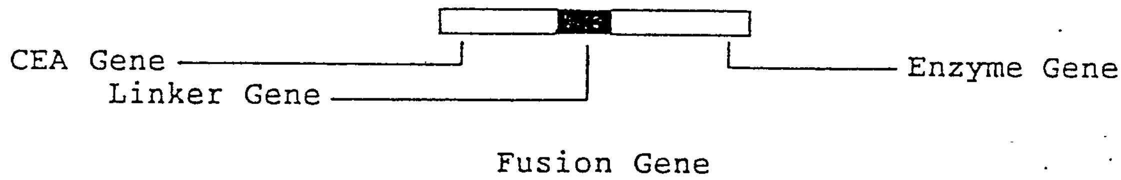


DIAGRAM 3

The fusion gene is constructed by ligation of the component genes in known manner. The linker may be synthesized directly into the CEA gene. Appropriate restriction sites may be engineered into the fusion gene.

The fusion gene is put into a PSV2 vector and electroporated into Sp210 and expressed. The product is screened for CEA and enzyme activity.

A typical EIA for CEA using two MABs is described by Hedin, et al., PNAS 80:3470-3474 (1983):

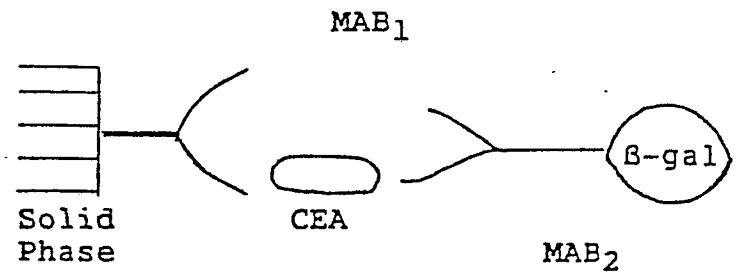


DIAGRAM 4

Pursuant to this invention the Hedin assay may be modified to provide an inhibition assay shown by Diagram 5:

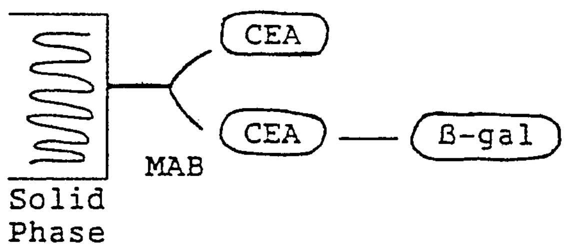


DIAGRAM 5

As Diagram 5 shows, the modified assay utilizes a single monoclonal antibody bound to a solid phase. The enzyme fusion product is CEA-β-galactosidase. The immonoreaction between the fusion product and the MAB is a direct measure of binding but is inhibited by free CEA. The greater the inhibition, the greater the amount of the free CEA in the sample analyzed. Specific steps may include (i) addition of a sample plus CEA-β-galactosidase fusion product to the MAB-solid phase reagent followed by incubation for 30-60 minutes; (ii) removal of the unbound material by washing; (iii) incubation with the substrate, e.g., O-nitrophenyl-β-galactoside; and (iv) observing the result in comparison of the result to a standard inhibition curve.

## Claims

1. A fragment of a CEA molecule including CEA domain III as depicted in Diagram 1C or any portion thereof coding for a CEA specific epitope.
2. A fragment of a CEA molecule including CEA domain IIIA, but not CEA domain IIIB as depicted in Diagram 1C or any portion thereof coding for a CEA specific epitope.
3. A fragment of a CEA molecule including CEA domain IIIB, but not CEA domain IIIA as depicted in Diagram 1C or any portion thereof coding for a CEA specific epitope.
4. A purified natural DNA Sequence I or any portion thereof coding for a CEA specific epitope.
5. A purified natural DNA sequence coding for CEA subdomain IIIA as depicted by Sequence I or any portion thereof coding for a CEA specific epitope.
6. A purified natural DNA sequence coding for CEA subdomain IIIB as depicted by Sequence I or any portion thereof coding for a CEA specific epitope.
7. A synthetic peptide having the sequence depicted by Sequence I or any portion thereof coding for a CEA specific epitope.
8. A synthetic peptide having the sequence depicted by Sequence I for CEA subdomain IIIA or any portion thereof coding for a CEA specific epitope.
9. A peptide having the sequnce depicted by Sequence I for CEA subdomain IIIB of any portion thereof coding for a CEA specific epitope.
10. A synthetic DNA sequence coding for CEA domain III as depicted by Sequence I or any portion thereof coding for a CEA specific epitope.
11. A synthetic DNA sequence coding for CEA domain IIIA but not CEA domain IIIB as depicted by Sequence I or any portion thereof coding for a CEA specific epitope.
12. A synthetic DNA sequence coding for CEA domain IIIB but not CEA domain IIIA as depicted by Sequence I or any portion thereof coding for a CEA specific epitope.
13. A fusion product of an enzyme of any fragment of a CEA molecule as defined by any one of claims 1, 2 and 3.
14. A fusion product of an enzyme and a DNA sequence as defined by any one of claims 4, 5, 6, 10, 11 and 12.
15. A synthetic nucleotide sequence corresponding to CEA domain IIIA as depicted by Sequence I linked by a restriction enzyme cleavage site to a synthetic nucleotide sequence corresponding to CEA domain IIIB as depicted by Sequence I.
16. A synthetic peptide comprising amino acid Sequences II, III, IV, V, VI or VII.
17. A CEA fragment having topology as depicted by one of Figures 2A, 2B, 3A, 3B, 4A or 4B.

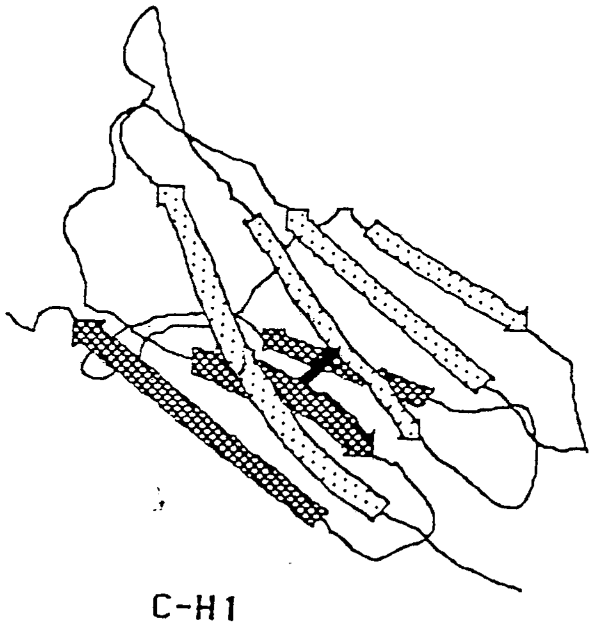

C-H1

FIGURE 1

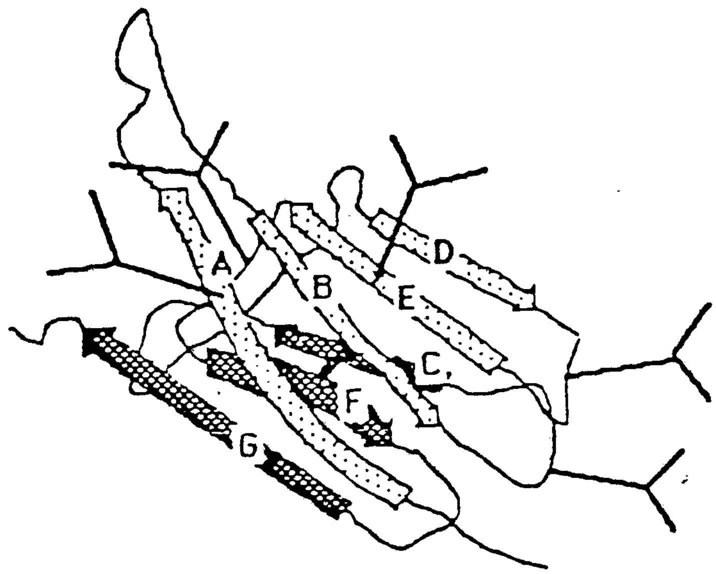


FIGURE 2A

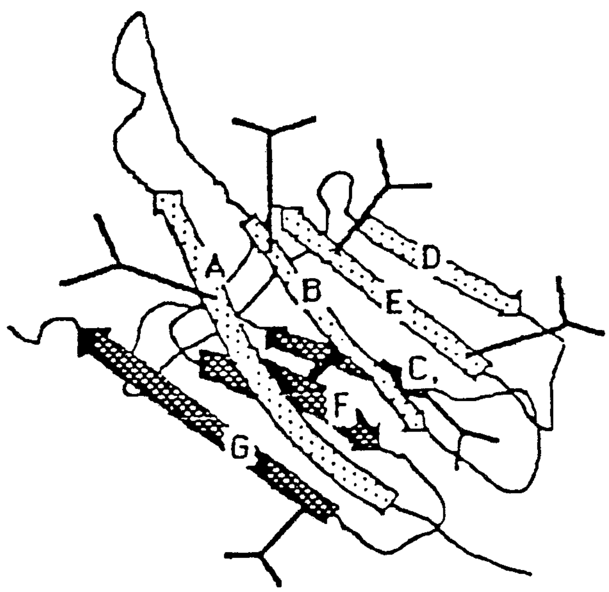


FIGURE 2B

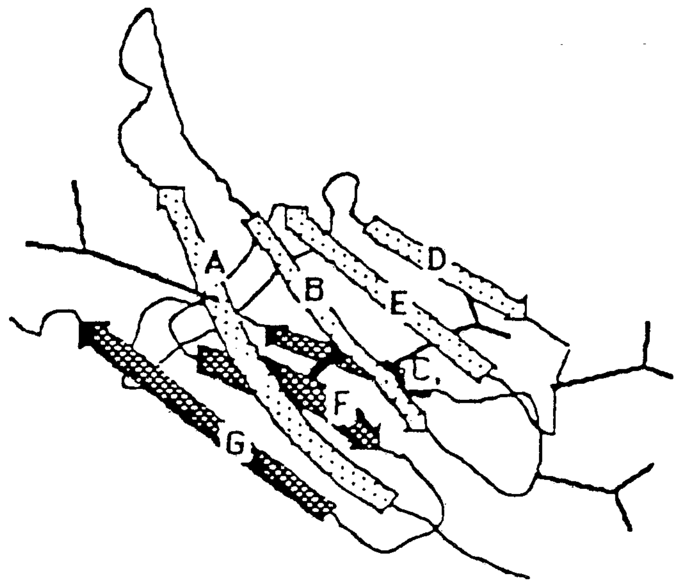


FIGURE 3A

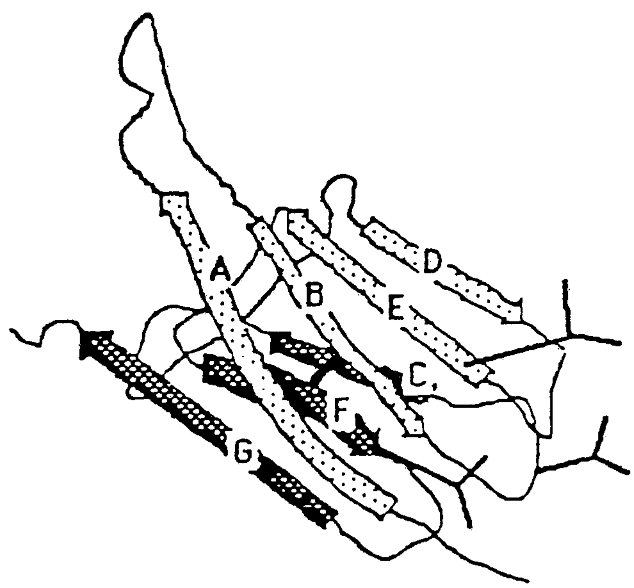


FIGURE 3B

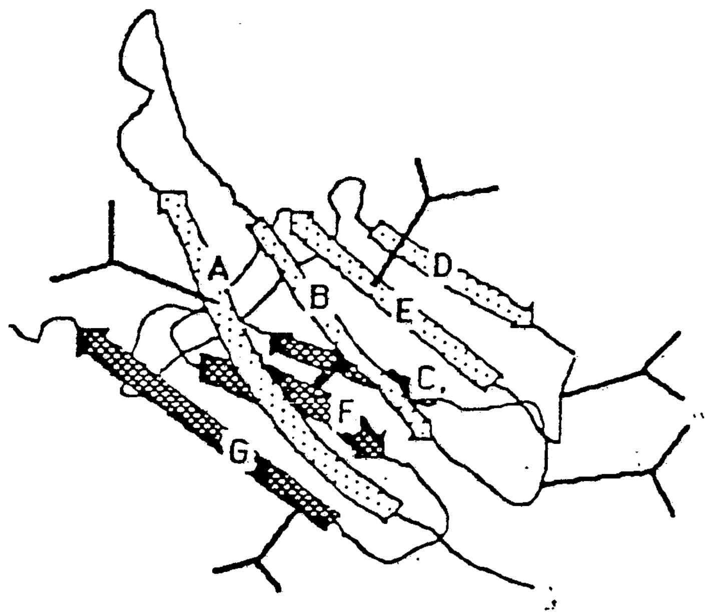


FIGURE 4A

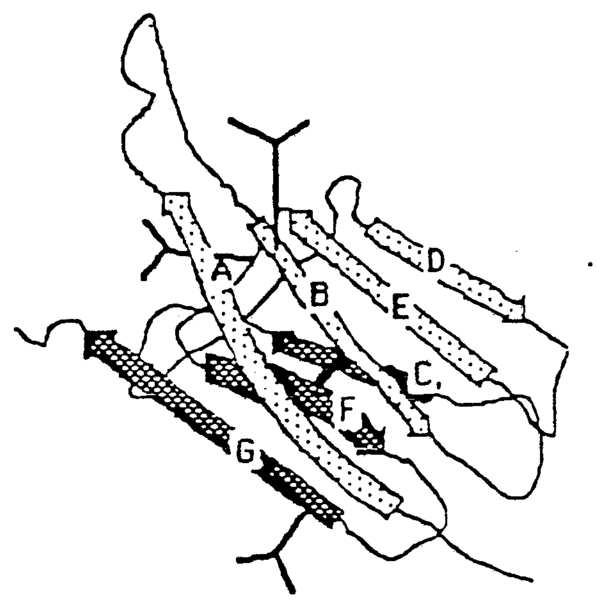


FIGURE 4B